# EUROPÄISCHE PATENTSCHRIFT

(11) **EP 1 071 951 B1**
(45) Veröffentlichungstag und Bekanntmachung des Hinweises auf die Patenterteilung: **09.10.2002**
(21) Anmeldenummer: 99916917.0
(22) Anmeldetag: 13.04.1999
(51) Int. Cl.: G01N 33/53, G01N 33/48

(54) **MODIFIZIERTE OBERFLÄCHE FÜR DIE DURCHFÜHRUNG ODER DEN NACHWEIS VON AFFINITÄTSREAKTIONEN**
MODIFIED SURFACE FOR CARRYING OUT OR DETECTING AFFINITY REACTIONS
SURFACE MODIFIEE DESTINEE A L'EXECUTION OU A LA DETECTION DE REACTIONS D'AFFINITE

(30) Priorität: 15.04.1998 DE 19816604
(43) Veröffentlichungstag der Anmeldung: 31.01.2001
(73) Patentinhaber: Bodenseewerk Perkin-Elmer Gmbh, 88662 Überlingen (DE)
(72) Erfinder: GAUGLITZ, Günter, D-72070 Tübingen (DE); BRECHT, Andreas, D-73252 Lenningen (DE); PIEHLER, Jacob, D-72070 Tübingen (DE)
(74) Vertreter: Pfau, Anton Konrad, Dr.
(86) Internationale Anmeldenummer: EP9902485
(87) Internationale Veröffentlichungsnummer: WO99053317

(56) Entgegenhaltungen:
- BRECHT, A. ET AL.: "A direct optical immunosensor for atrazine detection" ANALYTICA CHIMICA ACTA, Bd. 311, 1995, Seiten 289-299, XP002900571
- LÖFAS, S.: "Dextran modified self-assembled monolayer surfaces for use in biointeraction analysis with surface plasmon resonance" PURE & APPL. CHEM., Bd. 67, Nr. 5, 1995, Seiten 829-834, XP002900572
- GAUGLITZ, G. ET AL.: "Chemical and biochemical sensors based on interferometry at thin (multi-) layers" SENSORS AND ACTUATORS, Bd. B11, 1993, Seiten 21-27, XP002900573

## Beschreibung

Die Erfindung betrifft eine Einrichtung für die Durchführung und/oder den Nachweis von Affinitätsreaktionen, insbesondere eine Sondeneinrichtung, mit mindestens einer Oberfläche oder Grenzfläche, an der eine Affinitätsreaktion stattfindet, und mindestens einem an die Oberfläche gebundenen Polymer.

Hintergrund der Erfindung ist u.a. das Bestreben, Affinitätsreaktionen, insbesondere biochemische Affinitätsreaktionen mit optischen, akustischen oder anderen physikalischen Methoden "markierungsfrei", d.h. ohne Verwendung entsprechender Markierungssubstanzen, zu detektieren. Als physikalische Nachweismethode sind dabei insbesondere optische Verfahren geeignet, wie die Oberflächenplasmonenresonanz, die reflektometrische Interferenzspektroskopie, die frustrierte Totalreflexion, Gitterkoppler und die sog. integriert-optischen Verfahren.

Voraussetzung für eine derartige markierungsfreie Detektion ist die selektive Wechselwirkung der Moleküle, insbesondere der Biomoleküle an die Oberfläche bzw. Grenzfläche eines physikalischen Signalwandlers. Zu diesem Zweck muß eine der miteinander wechselwirkenden Substanzen an der Oberfläche in definierter Form immobilisiert werden. Diese Ankopplung muß ausreichend stabil sein, um eine lange Lebensdauer, gerade auch unter ungünstigen Bedingungen wie chemisch aggressiven Bedingungen zu gewährleisten. Außerdem muß sichergestellt sein, daß an die Oberfläche keine Moleküle unspezifisch adsorbiert werden, sondern nur spezifisch solche Moleküle-, deren Wechselwirkung mit den angekoppelten Substanzen detektiert werden soll. Dieser Anforderung kommt bei einer markierungsfreien Detektion besondere Bedeutung zu, da die dort verwendeten Nachweismethoden jegliche Veränderung an der Oberfläche nachweisen und daher nicht zwischen spezifischer und unspezifischer Bindung unterscheiden können.

Übliche Signalwandler-Oberflächen, die beispielsweise aus Gold, Quarz oder Metalloxiden bestehen, können die genannten Anforderungen nicht erfüllen. Es ist deshalb bereits bekannt, derartige Oberflächen gezielt zu modifizieren. Dabei muß allerdings gewährleistet sein, daß eine derartige Modifizierung keinen negativen Einfluß auf die Detektion selbst, beispielsweise deren Empfindlichkeit nimmt. Bei optischen Nachweisverfahren werden daher üblicherweise zur Modifizierung sehr dünne Oberflächenschichten, vorzugsweise < 50 nm, einheitlichen Aufbaus und hoher Dichte angestrebt. Dies gilt insbesondere für optische Nachweisverfahren, die auf gerichteter Reflexion beruhen, wie z.B. interferometrische oder ellipsometrische Verfahren, aber auch für Totalreflexion, wenn die Eindringtiefe des evaneszenten Feldes gering und daher die Empfindlichkeit mit der Entfernung von der Oberfläche stark abnimmt, z.B. beim Gitterkoppler.

So lassen sich auf Gold oder Quarz unverzweigte, langkettige Alkylgruppen unter Ausbildung einer Monolage kovalent anbinden und zur Immobilisierung biochemischer Komponenten verwenden, sh. beispielsweise J. Rickert et al. in Biosensors & Bioelectronics Vol. 11, No. 6/7, pp. 591-598, 1996. Hierbei handelt es sich jedoch um einen passiven Effekt, der nur schwer gezielt beeinflußbar ist. Hier erfolgt die Abschirmung rein durch Abdeckung unter Bildung einer dichten Schicht von Ketten, die miteinander wechselwirken. Man spricht von sog. self-assembling monolayers (SAM).

Weiter wurde die Anbindung von Polyethylenglykol und anderer Polymere an Oberflächen im Hinblick auf deren Verwendbarkeit zur Immobilisierung chemischer und biochemischer Substanzen bereits untersucht. Die Veröffentlichung von J. Piehler et al. in Biosensors & Bioelectronics Vol. 11, No. 6/7, pp. 579-590, 1996 zeigt jedoch, daß keine effiziente Abschirmung der Oberfläche erreicht werden kann. Dies dürfte auf eine zu geringe Dichte der kovalent angekoppelten Polymerketten zurückzuführen sein.

Deshalb ist im Bereich der markierungsfreien Detektion von Affinitätsreaktionen weiterhin die kovalente Anbindung von Dextranschichten mit Hydrogel-Eigenschaften verbreitet (siehe S. Löfas in Pure Appl. Chem. 67 (1995), 829-834). Bei einer derartigen Oberflächenmodifizierung sorgt zwar die hohe Hydrophilie der Dextran-Polymere für eine geringe Adsorption von Proteinen und damit für eine normalerweise geringe unspezifische Bindung. Allerdings ist eine Abschirmung gegen die unspezifische Bindung von Makromolekülen nicht grundsätzlich gewährleistet. Außerdem hat eine Modifizierung der Oberfläche mit Dextranschichten den Nachteil, daß die Ankopplung der immobilisierten Komponente nicht definiert an einer Oberfläche erfolgt, sondern statistisch innerhalb der Hydrogelmatrix von üblicherweise 50 bis 200 nm Durchmesser. Damit ist die Zugänglichkeit der immobilisierten Komponente für die mit ihr wechselwirkende Substanz nicht definiert und ein erheblicher Einfluß auf die Wechselwirkung festzustellen.

Die Erfindung stellt sich deshalb die Aufgabe, die aus dem Stand der Technik bekannten Nachteile zu vermeiden. Es soll insbesondere eine modifizierte Oberfläche für die Durchführung und/oder den Nachweis von Affinitätsreaktionen zur Verfügung gestellt werden, an der unspezifische Wechselwirkungen effizient unterdrückt sind. Die an der Oberfläche immobilisierten Substanzen sollen unter guter Zugänglichkeit in Form dünner Schichten stabil angekoppelt oder ankoppelbar sein, so daß derartige modifizierte Oberflächen eine lange Lebensdauer und eine gute Regenerierbarkeit aufweisen. Die Oberflächen sollen sich in besonderer Weise zur markierungsfreien Detektion von Affinitätsreaktionen, insbesondere biochemischen Affinitätsreaktionen, mit optischen Nachweismethoden eignen.

Diese Aufgabe wird gelöst durch die Einrichtung mit den Merkmalen des Anspruchs 1 und durch das Verfahren mit den Merkmalen des Anspruchs 19. Bevorzugte Ausführungsformen dieser Einrichtung und dieses Verfahrens sind in den abhängigen Ansprüchen 2 bis 18 bzw. 20 bis 23 dargestellt. Der Wortlaut sämtlicher Ansprüche wird hiermit durch Bezugnahme zum Inhalt dieser Beschreibung gemacht.

Erfindungsgemäß ist die eingangs genannte Einrichtung dadurch erhältlich, daß die Bindung des Polymers an die Oberfläche in reiner Phase, d.h. im wesentlichen lösemittelfrei, erfolgt. Durch die Anbindung in reiner Phase läßt sich offensichtlich eine modifizierte Oberfläche mit speziellen Eigenschaften zur Verfügung stellen, die diese gegenüber bekannten Oberflächen in besonderer Weise für die Durchführung oder den Nachweis von Affinitätsreaktionen geeignet macht. Die speziellen Eigenschaften der erfindungsgemäß modifizierten Oberfläche können darauf zurückzuführen sein, daß sich gegenseitig abstoßende Polymerketten eine sehr dünne Oberflächenbeschichtung hoher Dichte und Orientierung bilden (Ausbildung eines dichten Polymer"rasens" nebeneinander angeordneter, sich ggf. gegenseitig abstoßender Polymermoleküle). Dies bewirkt eine effektive Abschirmung gegen die unspezifische Bindung jeglicher Moleküle. Damit ist die modifizierte Oberfläche in besonderer Weise zur Immobilisierung/Ankopplung einer (biochemischen) Substanz und damit zur Durchführung oder zum Nachweis einer entsprechenden Affinitätsreaktion mit dieser Substanz geeignet. Eine derartige modifizierte Oberfläche ist bei Verwendung von Lösungsmitteln zur Kupplung offensichtlich nicht realisierbar.

Bei der erfindungsgemäßen Einrichtung kann es sich insbesondere um eine Sondeneinrichtung mit einer eingangs erwähnten Signalwandler-Oberfläche handeln. Derartige Einrichtungen sind auch als sogenannte Biosensoren bekannt. Bei dem Begriff der "Bindung" im Sinne der Erfindung handelt es sich üblicherweise um eine kovalente Bindung.

Bei dem an die Oberfläche gebundenen Polymer handelt es sich insbesondere um ein weitgehend unverzweigtes Polymer, vorzugsweise mit kurzer Kettenlänge. Bei Verwendung unverzweigter Polymere läßt sich eine besonders dichte Anordnung der Polymerketten nebeneinander erreichen. Durch kurze Kettenlängen lassen sich dünne Schichten herstellen.

Es ist nach der Erfindung bevorzugt, wenn es sich bei dem beschriebenen Polymer um ein sog. hydrophiles Polymer handelt. Solche Polymere, für die im folgenden noch Beispiele genannt werden, können aufgrund ihres Ausschlußvolumens in wässrigem Medium Makromoleküle abstoßen. Diese grundsätzlich vorteilhafte Eigenschaft läßt sich bei der Erfindung besonders gut zur Geltung bringen.

Die Molmasse der Polymere ist grundsätzlich frei wählbar, beträgt jedoch vorzugsweise zwischen 200 und 50.000 g/mol, wobei Molmassen zwischen 200 und 5.000 g/mol weiter bevorzugt sind. In Anbetracht der nicht eindeutig definierten Grenze zwischen Oligomeren und Polymeren können Polymere niederer Molmasse im Sinne der Erfindung auch als Oligomere bezeichnet werden. Innerhalb der genannten bevorzugten Molmassenbereiche sind Molmassen zwischen 200 und 20.000 g/mol und innerhalb dieses Bereiches wiederum Molmassen zwischen 500 und 5.000 g/mol hervorzuheben. Den genannten Polymermassen können Kettenlängen des gestreckten Polymers von 2 bis 20 nm, vorzugsweise 2 bis 10 nm entsprechen.

Weiter besitzt das Polymer bei der Erfindung insbesondere einen Schmelzpunkt von < 200 °C, vorzugsweise von < 100 °C. Wie bereits beschrieben, erfolgt bei der Erfindung die Bindung des Polymers an die Oberfläche in reiner Phase. Es ist deshalb bevorzugt, wenn das Polymer in flüssiger Form vorliegt oder in einfacher Weise in die flüssige Phase überführbar ist. Dies erfolgt vorzugsweise durch Schmelzen, so daß ein bei vergleichsweise niedrigen Temperaturen (< 200 °C, besser < 100 °C) schmelzbares Polymer erfindungsgemäß besonders gut einsetzbar ist.

In Weiterbildung besitzt das Polymer endständige funktionelle Gruppen, wobei grundsätzlich monofunktionalisierte Polymere einsetzbar sind. Vorzugsweise handelt es sich jedoch um endständig homo- oder heterosubstituierte bifunktionelle Polymere, so daß eine endständige Funktion für die Anbindung des Polymers an die Oberfläche und die andere endständige Funktion für die Anbindung einer wechselwirkenden Substanz (Ligand) zur Verfügung steht.

Bei den am Polymer vorhandenen funktionellen Gruppen kann es sich um NH₂, OH-, SH-, COOH-, OCH₃-, Oxiran-, SCN- und/oder OCN-Gruppen handeln, wobei NH₂, OH- und/oder COOH-Gruppen bevorzugt sind.

Erfindungsgemäß können ohne weiteres Mischungen verschiedener Polymere oder auch Copolymere eingesetzt werden. Bei solchen Mischungen kann es sich beispielsweise um Mischungen von Polymeren, die sich lediglich in ihrer Molmasse unterscheiden, oder um Mischungen von Polymeren, die unterschiedliche funktionelle Gruppen tragen, handeln. Im letzteren Fall stehen ggf. verschiedene funktionelle Gruppen für die Ankopplung von wechselwirkenden Substanzen zur Verfügung.

Bei bevorzugten Ausführungsformen der Erfindung handelt es sich bei dem Polymer um ein Polyalkylenglykol. Von den typischen Vertretern der Polyalkylenglykole, nämlich Polymethylenglykol, Polyethylenglykol und Polypropylenglykol, ist die Verwendung von Polyethylenglykol bevorzugt. Als bevorzugte Polyethylenglykole (PEG) sind die homosubstituierten bifunktionellen Substanzen Diamino-PEG und Dicarboxy-PEG zu nennen, wobei jeweils Molmassen zwischen 200 g/mol und 20.000 g/mol bevorzugt sind, vorzugsweise Molmassen von ca. 2.000 g/mol und 20.000 g/mol, insbesondere zwischen 2.000 und 5.000 g/mol. Es können selbstverständlich auch Copolymerisate verschiedener Polyalkylenglykole oder Mischungen verschiedener Polyalkylenglykole verwendet werden.

Obwohl mit den erwähnten Polyalkylenglykolen bei der Erfindung besonders gute Ergebnisse erzielt werden, ist die Erfindung nicht auf die Verwendung dieser Polymere beschränkt. Grundsätzlich lassen sich alle Polymere, die in reiner Phase umsetzbar sind, verwenden, insbesondere die hydrophilen Polymere mit hohem Ausschlußvolumen und entsprechender Funktionalisierung. Als besonders geeignete Polymere lassen sich die Polyalkylenimine nennen, wobei hier insbesondere das Polyethylenimin hervorzuheben ist. Weitere Beispiele sind terminal funktionalisierte Polyvinylalkohole, Polyhydroxyethylmethacrylate und Polyamide.

Wie bereits dargestellt, befindet sich die Oberfläche, an die das Polymer bei der Erfindung gebunden ist, an einem geeigneten Träger oder einem geeigneten Substrat, das die Einrichtung oder einen Teil der Einrichtung bildet. Vorzugsweise ist die Oberfläche an einem Signalwandler oder Biosensor ausgebildet. Die Oberfläche ist dabei vorzugsweise planar.

Vor Anbindung des Polymers kann die Oberfläche bei der Erfindung freie OH-Gruppen aufweisen, da derartige Gruppen einen guten Ausgangspunkt für eine Anbindung bilden.

Die Oberfläche oder auch der Träger selbst kann von einem Metall, beispielsweise Gold und/oder Silber, oder einem Metalloxid gebildet sein, wobei es sich vorzugsweise um Siliciumdioxid handelt. Als weitere bevorzugte Oberflächen-/Trägermaterialien sind auch Glas, Titandioxid (TiO₂), Tantaloxid (Ta₂O₅) und Indium-Zinnoxid (ITO) zu nennen.

Bei weiter bevorzugten Ausführungsformen der Erfindung wird die Oberfläche vor der Anbindung des Polymers modifiziert/aktiviert, wobei bei dieser Aktivierung vorzugsweise funktionelle Gruppen eingeführt werden. Diese funktionellen Gruppen können dann direkt oder mit Hilfe zusätzlicher Kupplungsreagenzien mit funktionellen Gruppen des Polymers umgesetzt und auf diese Weise das Polymer angebunden werden. Die Einführung funktioneller Gruppen an die Oberfläche ist dann angezeigt, wenn die Oberfläche selbst keine befriedigende Umsetzung mit dem Polymer erlaubt. Bei Metallen als Oberfläche/Träger kann die Funktionalisierung durch Alkylthiole erfolgen. Hier handelt es sich vorzugsweise um Alkylthiole der allgemeinen Formeln HS-(CH₂)ₙY oder (S-(CH₂)ₙY)₂ mit einer geeigneten funktionellen Gruppe, insbesondere einer Epoxygruppe als Gruppe Y und mit n zwischen 1 und 15. Die Einführung der funktionellen Gruppen an die Oberfläche bei Metalloxiden erfolgt insbesondere durch Silanisierung, wobei vorzugsweise Silanverbindungen der allgemeinen Formel X-Si-R-Y verwendet werden können. Derartige Silanverbindungen werden über die mit X bezeichnete Gruppe kovalent an die Oberfläche angebunden. Bei dem Rest X kann es sich vorzugsweise um einen Alkoxy-Rest oder einen Halogen-Rest, insbesondere einen Chlorrest, handeln. Bei der Gruppe R handelt es sich üblicherweise um eine Alkylkette, die vorzugsweise unverzweigt ist und durch Heteroatome unterbrochen sein kann. Die Kettenlänge der Gruppe R kann üblicherweise zwischen 4 und 30 Atomen betragen. Bei der Gruppe Y handelt es sich um eine geeignete funktionelle Gruppe, vorzugsweise eine Epoxy-Gruppe.

Bei den eingeführten funktionellen Gruppen kann es sich vorzugsweise um NH₂-, OH-, SH-, Ester-, Oxiran-, SCN- und/oder OCN-Gruppen handeln. Beim Einsatz von Ester-Gruppen sind sogenannte aktivierte Ester, d.h. besonders reaktionsfähige Estergruppen, bevorzugt.

Wie bereits erwähnt, werden die an die Oberfläche eingeführten funktionellen Gruppen üblicherweise mit funktionellen Gruppen des Polymers zu dessen Ankopplung umgesetzt. Aus der folgenden Liste ergeben sich besonders geeignete Kombinationen der funktionellen Gruppen derartiger Reaktionspartner:

| Funktionelle Gruppe an der Oberfläche | Funktionelle Gruppe am Polymer |
|---|---|
| Oxiran-, Aktivester-, SCN-, OCN-Gruppe | NH₂-, OH-, SH-Gruppe |
| | |
| Oxirangruppe | COOH-Gruppe |
| | |
| NH₂-, OH-, SH-Gruppe | Oxiran-, SCN-, OCN-Gruppe |
| | |
| OH-Gruppe (aktiviert mit Divinylsulfon, Carbonyldiimidazol) | OH-Gruppe |
| | |
| OH-Gruppe (aktiviert mit Tosylchlorid, Tresylchlorid, Cyanurchlorid, Divinylsulfon) | NH₂-Gruppe |

Wie bereits beschrieben, wird das Polymer erfindungsgemäß bevorzugt als flüssiges oder geschmolzenes Polymer zur Bindung an die Oberfläche eingesetzt. Dabei findet erfindungsgemäß noch keine Zersetzung der an der Reaktion beteiligten Komponenten statt. Dementsprechend sind Polymere mit einem Schmelzpunkt < 200 °C, vorzugsweise < 100 °C, bevorzugt. Übliche und bevorzugte Temperaturen für die Bindung des Polymers an die Oberfläche liegen dementsprechend zwischen ca. 50 °C und ca. 100 °C, wobei innerhalb dieses Bereichs Temperaturen zwischen 50 °C und 80 °C hervorzuheben sind. Eine übliche Umsetzungstemperatur beträgt beispielsweise ca. 60 °C.

Um eine Bindung des Polymers an die Oberfläche in reiner Phase zu erreichen, können längere Reaktionszeiten bevorzugt sein. So kann die Umsetzung innerhalb eines Zeitraums von einigen Stunden bis einigen (mehreren) Tagen erfolgen. Übliche Umsetzungszeiten liegen zwischen 12 und 60 Stunden. Um eine zuverlässige Anbindung zu erreichen, ist es häufig zweckmäßig und bei geringerer Reaktivität der Komponenten bevorzugt, höhere Reaktionstemperaturen und/oder längere Reaktionszeiten zu wählen. Dies ist insbesondere dann erfolgversprechend, wenn als funktionelle Gruppen sowohl an der Oberfläche als auch am Polymer OH-Gruppen und COOH-Gruppen gewählt werden.

Die erfindungsgemäße Einrichtung ist weiter dadurch gekennzeichnet, daß an dem an die Oberfläche gebundenen Polymer ein Ligand, insbesondere ein biochemisch aktiver Ligand immobilisiert ist. Dieser üblicherweise über die zweite funktionelle Gruppe am Polymer angekoppelte Ligand steht dann für die eigentliche Affinitätsreaktion zur Verfügung, bei der die nachzuweisende Komponente mit dem immobilisierten Liganden wechselwirkt. Die Immobilisierung des Liganden kann in üblicher Weise mit Hilfe üblicher Reaktionen erfolgen, wie dies im folgenden anhand von Beispielen noch erläutert wird.

Die Erfindung umfaßt weiter eine Oberflächenbeschichtung für Oberflächen und Träger, die zur Durchführung oder für den Nachweis von Affinitätsreaktionen eingesetzt werden. Die Merkmale einer derartigen Oberflächenbeschichtung gehen aus dem bisherigen Text der Beschreibung hervor.

Wie bereits erläutert, besitzt die erfindungsgemäß modifizierte Oberfläche bzw. die Oberflächenbeschichtung gegenüber dem Stand der Technik entscheidende Vorteile. So wird durch die Anbindung des Polymers an die Oberfläche in reiner Phase eine dichte und festgebundene dünne Schicht hoher Stabilität zur Verfügung gestellt, die nach Immobilisierung eines geeigneten Liganden eine hochspezifische Bindung der nachzuweisenden bzw. umzusetzenden Spezies ermöglicht. Eine unspezifische Bindung anderer Substanzen wird weitgehend ausgeschlossen. Die modifizierte Oberfläche hat eine hohe Lebensdauer und eine gute. Regenerierbarkeit. Alle diese vorteilhaften Eigenschaften machen die modifizierte Oberfläche in besonderer Weise für ihren Einsatz bei Biosensoren oder Biosonden geeignet. Biochemische Wechselwirkungen an Grenzflächen können mit Hilfe der bereits genannten, insbesondere der optischen Methoden markierungsfrei detektiert werden.

Weiter umfaßt die Erfindung ein Verfahren zur Herstellung einer Einrichtung mit modifizierter Oberfläche, die zur Durchführung oder zum Nachweis von Affinitätsreaktionen vorgesehen ist. Dabei wird mindestens ein Polymer an eine Oberfläche, an der eine Affinitätsreaktion stattfinden soll, in reiner Phase, d.h. im wesentlichen ohne Einsatz eines Lösungsmittels, gebunden. Bezüglich der vorzugsweise verwendbaren Polymere, der vorzugsweise verwendbaren Oberflächen und der bevorzugten Verfahrensparameter wird auf die bisherige Beschreibung verwiesen und ausdrücklich Bezug genommen.

Schließlich umfaßt die Erfindung die Verwendung der beschriebenen Einrichtung zur markierungsfreien Detektion von Affinitätsreaktionen, insbesondere von biochemischen Affinitätsreaktionen. Als Nachweisverfahren wird dabei vorzugsweise ein optisches Verfahren, insbesondere die sogenannte reflektometrische Interferenzspektroskopie eingesetzt.

Die beschriebenen Merkmale und weitere Merkmale der Erfindung ergeben sich aus der nachfolgenden Beschreibung von bevorzugten Ausführungsformen in Verbindung mit den Unteransprüchen, Beispielen und den Zeichnungen. Hierbei können die einzelnen Merkmale jeweils für sich oder zu mehreren in Kombination miteinander verwirklicht sein. In den Zeichnungen zeigen:
- Fig. 1: die zeitliche Änderung der optischen Schichtdicke bei der Untersuchung einer ersten erfindungsgemäß modifizierten Oberfläche auf spezifische und unspezifische Wechselwirkung,
- Fig. 2a: die zeitliche Änderung der optischen Schichtdicke bei der Untersuchung einer zweiten erfindungsgemäß modifizierten Oberfläche auf unspezifische Wechselwirkung,
- Fig. 2b: die zeitliche Änderung der optischen Schichtdicke bei der Untersuchung einer zweiten erfindungsgemäß modifizierten Oberfläche auf spezifische Wechselwirkung, und
- Fig. 3: die zeitliche Änderung der optischen Schichtdicke bei der Untersuchung einer erfindungsgemäß modifizierten Oberfläche und von zwei Referenzoberflächen auf unspezifische Wechselwirkung.

### Beispiel 1

Zur Herstellung einer modifizierten Oberfläche wird ein Glasträger vorgelegt, auf den durch einen Sputterprozeß eine SiO₂-Schicht in einer Dicke von 300 bis 400 nm aufgebracht wurde. Dieser Glasträger mit SiO₂-Schicht wurde zunächst wie folgt vorbehandelt:
- Abreiben grober Verunreinigungen mit einem Papiertuch,
- Inkubation mit 6 N NaOH während 2 min und anschließendes Abspülen mit Wasser,
- Inkubation mit einem frisch hergestellten Gemisch aus konz. H₂SO₄ und konz. H₂O₂ (3:2) während 30 min und anschließendes Abspülen mit Wasser, und
- Trocknen der Oberfläche bei Raumtemperatur an Luft.

Die so vorbehandelte Oberfläche wurde anschließend mit Glycidyloxipropyltrimethoxysilan (GOPTS), bezogen von Fluka, Deisenhofen, aktiviert. Dabei wurde wie folgt vorgegangen:
- Inkubation von 10 µl/cm² reines GOPTS auf die Oberfläche während 1 Std. bei Raumtemperatur, und
- anschließendes Abspülen mit trockenem Aceton und Trocknung mit Stickstoff.

Unmittelbar anschließend an die Aktivierung der Oberfläche durch Silanisierung mit GOPTS wurde ein bifunktionelles Polyethylenglykol (PEG), nämlich ein Diamino-PEG mit Molmasse 2.000 g/mol (Produkt DAPEG 2000 der Fa. Rapp Polymere, Tübingen), an die aktivierte Oberfläche gebunden. Die Umsetzung ging dabei wie folgt vonstatten:
- Aufbringen von 5 mg/cm³ Polyethylenglykol auf die Oberfläche und Schmelzen des Polyethylenglykols bei ca. 60 °C bis ca. 70 °C in einem Ofen,
- Auflegen eines Glasplättchens auf die Oberfläche zur gleichmäßigen Verteilung des geschmolzenen Polymers,
- Temperaturbehandlung/Umsetzung im Ofen bei ca. 60 °C während 36 Std., und
- Abspülen des überschüssigen Polyethylenglykols mit Wasser und Trocknen an Luft bei Raumtemperatur.

Nach dieser Umsetzung ist das Polyethylenglykol (Polymer) kovalent an die Oberfläche gebunden bzw. gekuppelt. Die zweite Aminogruppe des bifunktionellen Polyethylenglykols steht für die Immobilisierung einer chemischen oder biochemischen Komponente zur Verfügung, die selbst wiederum an einer Affinitätsreaktion teilnehmen kann.

Im vorliegenden Fall wurde an die endständige freie Aminogruppe ein Carboxyl-funktionalisiertes Triazin-Derivat, nämlich 4-Chloro-6-(isopropylamino)-1,3,5-Triazin-2-(6'amino)-capronsäure (CTCA) gekuppelt. Dabei wurde wie folgt vorgegangen:
- Zugabe von 1 µl Diisopropylcarbodiimid (DIC) zu 10 µl einer Lösung von 1 mg CTCA pro 10 µl Dimethylformamid (DMF), (DIC und DMF von Fluka, Deisenhofen),
- Inkubation von 5 µl/cm² der so hergestellten Lösung auf die Oberfläche während mindestens 6 Std., und
- anschließendes Spülen der Oberfläche mit DMF und Wasser und Trocknung bei Raumtemperatur an Luft.

Zur funktionalen Charakterisierung der modifizierten Oberfläche mit dem immobilisierten Liganden CTCA wurde die unspezifische und die spezifische Bindung von Proteinen mit Hilfe der reflektometrischen Interferenzspektroskopie (RIfS) untersucht. Dabei wurden zur Inkubation die folgenden Lösungen verwendet:
a. 1 mg/ml Ovalbumin,
b. 100 µl/ml Kälberserum, und
c. 50 µg/ml eines für CTCA spezifischen Proteins, nämlich anti-Simazin Antikörper, Fab-Fragment.

Die reflektometrische Interferenzspektroskopie (RIfS) ist als Nachweisverfahren zur markierungsfreien Detektion von Affinitätsreaktionen bekannt. Die Grundlagen des Verfahrens sind beispielsweise in der Veröffentlichung von G. Gauglitz et al. in Sens. & Act., B 11, 21 bis 27 (1993) beschrieben. Es wird die Interferenz von Weißlicht bestimmt, das an dünnen Schichten reflektiert wird. Die Bindung von Proteinen an Oberflächen erhöht die optische Schichtdicke des Films, die als Änderung im Reflexionsspektrum detektiert wird (A. Brecht et al., Anal. Chim. Acta, 311, 289 bis 300 (1995)). Einzelheiten der im vorliegenden Fall verwendeten Apparatur können der bereits eingangs erwähnten Veröffentlichung von J. Piehler et al. in Biosensors & Bioelectronics Vol. 11, No. 6/7, 579-590, 1996 entnommen werden.

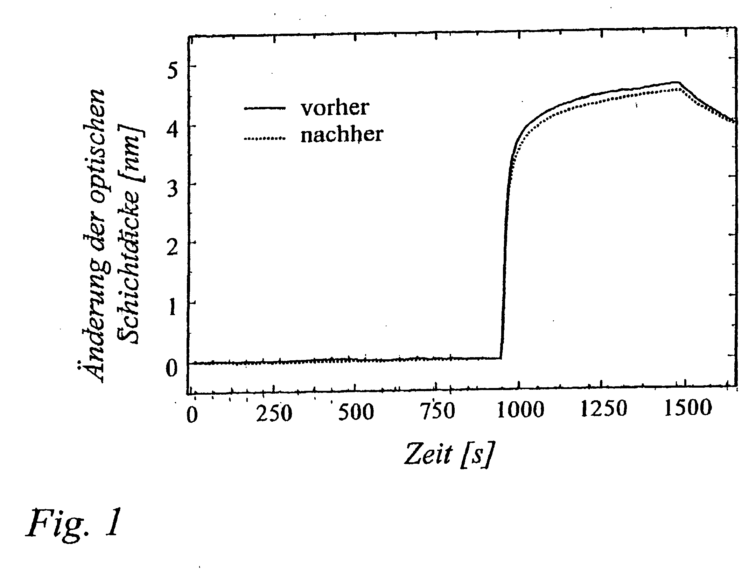

Die wesentlichen Ergebnisse der funktionalen Charakterisierung der gemäß Beispiel 1 hergestellten modifizierten Oberfläche sind in Fig. 1 dargestellt. Dabei ist die Änderung der optischen Schichtdicke in Abhängigkeit der Zeit aufgetragen. Außerdem wurde die Stabilität der Schichten untersucht, indem die funktionalen Eigenschaften nach 100 Regenerationen der Oberfläche mit 0,1 M HCl bestimmt wurde.

Fig. 1 zeigt zum einen, daß bei Inkubation mit Ovalbumin keine signifikante unspezifische Adsorption dieses Proteins detektiert werden konnte. Der Zeitraum der Ovalbumin-Inkubation ist in Fig. 1 durch den entsprechenden Zeitbalken dargestellt.

Bei der Inkubation mit Kälberserum wurde eine geringe unspezifische Bindung von 100 bis 200 pg/mm² gefunden, die vernachlässigbar und aus Gründen der Übersichtlichkeit in Fig. 1 nicht dargestellt ist.

Weiter zeigt Fig. 1, daß die spezifische Bindung von antisimazin Fab an das immobilisierte Triazin-Derivat sehr deutlich detektiert werden kann. Dabei entspricht die spezifische Bindung von etwa 4 bis 5 ng/mm² Fab der Menge, die für eine Monolage dieses Proteins zu erwarten ist. Es ist auch deutlich zu erkennen, daß die funktionalen Eigenschaften der modifizierten Oberfläche im Verlauf von 100 Regenerationen in hohem Ausmaß erhalten bleiben. Die spezifische Bindung verringert sich nach 100 Regenerationen um weniger als 5 %.

Die geschilderten Vorteile der Erfindung werden somit durch Beispiel 1 überzeugend untermauert.

Zur physikalischen Charakterisierung der modifizierten Oberfläche wurde parallel die Beladung der Oberfläche mit dem Polymer mit Hilfe von Spektralellipsometrie charakterisiert. Die Messungen mit dieser ebenfalls grundsätzlich bekannten Nachweismethode wurden an Luft durchgeführt. Aus Gründen der Methodik wurden in diesem Fall anstelle des Glassubstrats mit SiO₂-Schicht oxidierte Siliciumwafer (Wacker-Chemitronic, Burghausen) verwendet.

Die ellipsometrische Charakterisierung ergab im Fall von Beispiel 1 eine Oberflächenbeladung von fast 4 ng/mm². Dieser Wert liegt um einen Faktor 2 bis 4 über den mit anderen Methoden erreichbaren Beladungen. Er entspricht einer Oberflächenkonzentration von mehr als 10¹² Polymerketten/mm² bzw. einer Fläche von weniger als 1 nm² pro Polymerkette. Dies zeigt die dichte Packung der Polymerketten auf der Oberfläche bei der Erfindung.

### Beispiel 2

In der in Beispiel 1 beschriebenen Vorgehensweise wird ein Glassubstrat mit SiO₂-Schicht vorbehandelt, silanisiert und mit Polymer umgesetzt. Es wird lediglich anstelle des Diamino-PEGs ein anderes bifunktionelles Polyethylenglykol, nämlich Dicarboxy-PEG mit einer Molmasse von 2.000 g/mol (Produkt DCPEG 2000 der Fa. Rapp Polymere, Tübingen) verwendet.

An die verbleibenden freien Carboxyl-Gruppen wird anschließend ein amino-funktionalisierter Thrombininhibitor (TI) (BASF AG, Ludwigshafen) gekuppelt. Dazu wird wie folgt vorgegangen:
- Zugabe von 1 µl DIC zu 10 µl einer Lösung von 2 mg Thrombininhibitor pro 10 µl DMF,
- Inkubation von 5 µl/cm² der so hergestellten Lösung auf die Oberfläche während mindestens 6 Std., und
- anschließendes Abspülen der Oberfläche mit DMF und Wasser und Trockung bei Raumtemperatur an Luft.

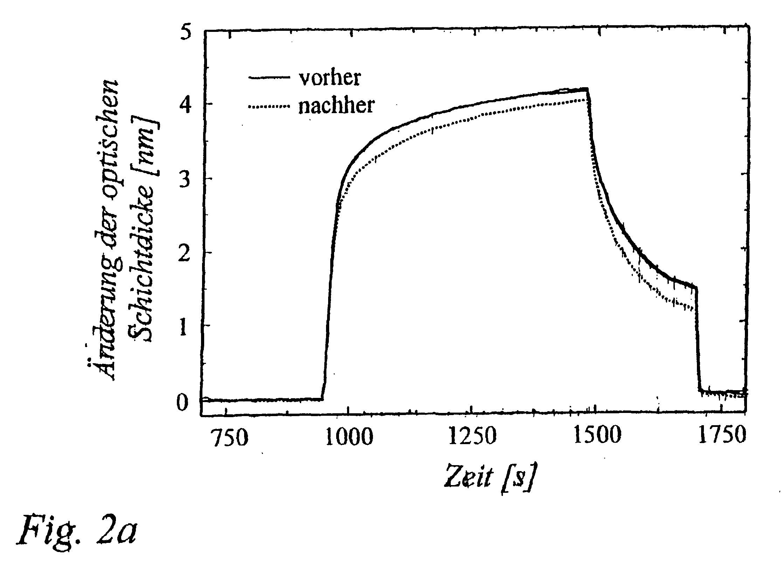

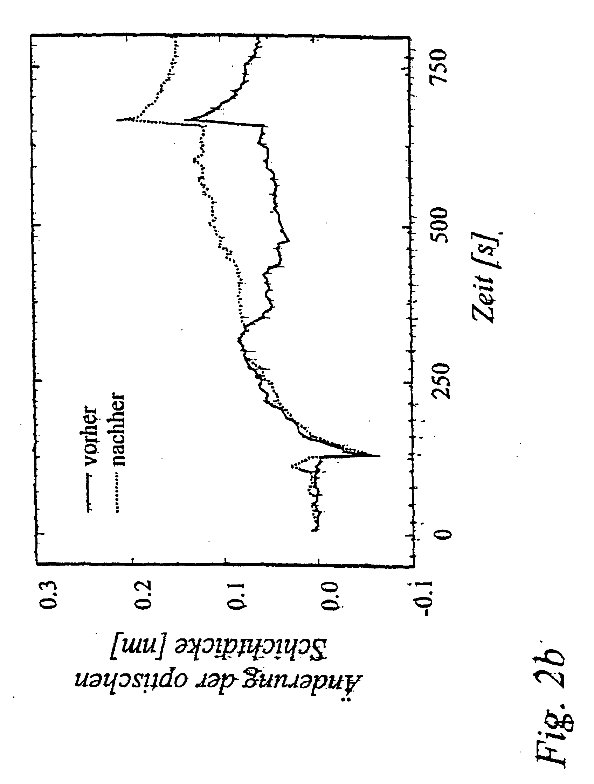

Auch im Beispiel 2 wird die funktionale und die physikalische Charakterisierung der modifizierten Oberfläche in der bereits in Beispiel 1 beschriebenen Weise vorgenommen. Die Ergebnisse sind in den Fig. 2a und 2b dargestellt, wobei jeweils die Änderung der optischen Schichtdicke über die Zeit aufgetragen ist.

Fig. 2a zeigt, daß bei einer Inkubation mit Kälberserum eine sehr schwache unspezifische Bindung von 100 bis 200 pg/mm² festgestellt werden kann. Die Dauer der Inkubation ist auch hier durch den Zeitbalken verdeutlicht. Eine wesentliche Beeinflussung dieser unspezifischen Bindung nach 100 Regenerationen mit 0,1 M HCl ist nicht zu erkennen.

Demgegenüber geht aus Fig. 2b deutlich hervor, daß die spezifische Bindung von Thrombin als spezifischem Protein für den immobilisierten Thrombininhibitor über die Änderung der optischen Schichtdicke deutlich nachweisbar ist. Auch hier deutet der erhaltene Wert auf die Bildung einer Monoläge hin. Bei Inkubation mit Ovalbumin konnte wiederum keine signifikante unspezifische Bindung an die Oberfläche detektiert werden. Letzteres ist in Fig. 2b aus Gründen der Übersichtlichkeit nicht dargestellt.

Die Untersuchung der Stabilität der funktionalen Eigenschaften nach 100 Regenerationen mit 0,1 M HCl ergibt ein vergleichbares Ergebnis wie bei Beispiel 1, nämlich daß sich die spezifische Bindung weniger als 5 % verringert.

Auch die an einem oxidierten Siliciumwafer mit Spektralellipsometrie erhaltenen Werte für die Oberflächenbeladung entsprechen den Ergebnissen von Beispiel 1.

### Beispiel 3

Es wird die spezifische Bindung und die unspezifische Bindung bei verschiedenen Polyethylenglykolen (PEGs) untersucht.

Zusätzlich zu den beiden in den Beispielen 1 und 2 verwendeten Polyethylenglykolen werden zusätzlich die folgenden Polymere eingesetzt:
- Methoxyamino-PEG mit Molmasse 2.000 g/mol (Produkt MAPEG 2000 der Fa. Rapp Polymere, Tübingen),
- Diamino-PEG mit Molmasse 20.000 g/mol (Produkt DAPEG 20000 der Fa. Rapp Polymere, Tübingen), und
- Dihydroxy-PEG mit Molmasse 3.000 g/mol (Produkt DHPEG 3000 von MERCK).

Die Herstellung der modifizierten Oberflächen erfolgt mit Hilfe der fünf verwendeten Polyethylenglykole wie in den Beispielen 1 und 2 beschrieben. Anschließend wird in der ebenfalls bereits beschriebenen Weise die unspezifische Bindung von Ovalbumin und Kälberserum sowie (teilweise) die spezifische Bindung untersucht, unter Einbeziehung der Ergebnisse der Beispiele 1 und 2. Zum Vergleich wird zusätzlich die unspezifische Bindung an einer reinen GOPTS-Schicht herangezogen.

Die Ergebnisse der Untersuchungen sind in der folgenden Tabelle zusammengefaßt:

| Polyethylenglykol | Ligand | Ovalbumin (1 mg/ml) in (pg/mm²) | Kälberserum (100 µl/ml) in (pg/mm²) | max.spezifische Bindung in (ng/mm²) |
|---|---|---|---|---|
| DAPEG 2000 | CTCA | < 10 | 100-200 | 4-5 |
| DAPEG 2000 | - | < 10 | 100-200 | - |
| DAPEG 20000 | CTCA | < 100 | 150-200 | 2,5 |
| DHPEG 3000 | - | < 10 | 150-200 | - |
| MAPEG 2000 | - | < 10 | < 100 | - |
| DCPEG 2000 | - | < 10 | 100-200 | - |
| DCPEG 2000 | TI | < 10 | 100-200 | 4-5 |
| | | | | |
| GOPTS | - | 100-300 | ca. 2000 | - |

Die Ergebnisse der Tabelle zeigen, daß im Vergleich zu einer reinen GOPTS-Schicht bei erfindungsgemäßer Verwendung der Polyethylenglykole in allen Fällen eine geringe unspezifische Bindung bzw. Wechselwirkung erreicht werden kann. Die Ergebnisse beim Polymer mit einer Molmasse von 20.000 g/mol lassen bei höheren Molmassen eine etwas höhere unspezifische Bindung erwarten. Im vorliegenden Versuch zeigt das Polyethylenglykol mit endständiger Methoxyfunktion eine etwas geringere unspezifische Bindung.

### Beispiel 4

Es wird die unspezifische Bindung aus einer löslichen Bakterienfraktion an einer erfindungsgemäß modifizierten Oberfläche mit zwei Referenzoberflächen verglichen. Dabei werden als Referenzoberflächen eine silanisierte Oberfläche und eine Oberfläche mit Polymer-Hydrogel auf Dextranbasis (Ausdehnung ca. 40 nm) ausgewählt.

Zur Herstellung der modifizierten Oberflächen wird wie folgt vorgegangen:
1. Die silanisierte Oberfläche wird wie in Beispiel 1 beschrieben hergestellt.
2. Für die Oberfläche mit Polymer-Hydrogel auf Dextranbasis wird zunächst ein Aminodextran mit einer Molmasse von 500.000 g/mol hergestellt, wie in der bereits genannten Veröffentlichung von J. Piehler et al. in Biosensors & Bioelectronics Vol. 11, No. 6/7, pp. 579-590, 1996 beschrieben.
   Dieses als AMD 500 bezeichnete Aminodextran wird an eine gemäß Beispiel 1 hergestellte GOPTS-modifizierte Oberfläche wie folgt gekuppelt:
   - Lösen von 100 mg AMD 500 in 300 µl Wasser und pH-Wert auf 7,5 einstellen,
   - 5 µl/cm² der so erhaltenen Lösung auf die silanisierte Oberfläche pipettieren und eine Glasplatte zur gleichmäßigen Verteilung auflegen,
   - 12 Stunden stehen lassen, anschließend mit Wasser abspülen und an Luft bei Raumtemperatur trocknen lassen.
3. Für die Herstellung der erfindungsgemäß modifizierten Oberfläche wird wie in Beispiel 1 (Verwendung von DAPEG 2000) verfahren.

Die zur Charakterisierung der unspezifischen Bindung aller drei Oberflächen verwendete Bakterienfraktion wird dadurch hergestellt, daß die lösliche Fraktion von 500 ml E. coli-Zellkultur (Stamm DH5) in 20 ml Puffer aufgenommen und mit Puffer auf 1/50 verdünnt wird.

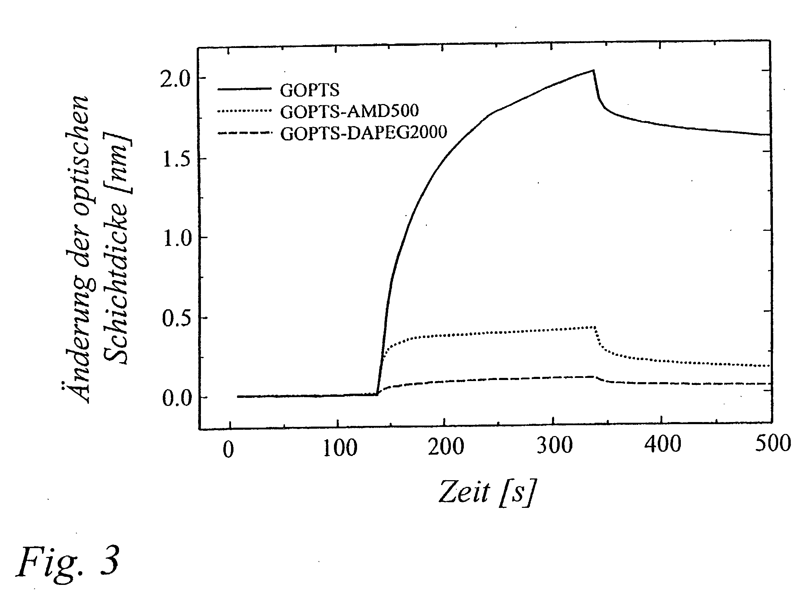

Die Untersuchungsergebnisse an den drei Oberflächen sind in Fig. 3 dargestellt. Es ist deutlich zu erkennen, daß die erfindungsgemäß modifizierte Oberfläche eine signifikant geringere unspezifische Bindung aufweist (maximal, ca. 100 pg/mm²) als die lediglich silanisierte Oberfläche (ca. 2 ng/mm²) und die mit Hydrogel modifizierte Oberfläche (ca. 400 pg/mm²). Dabei ist zu berücksichtigen, daß das erfindungsgemäß verwendete Polymer eine sehr viel geringere Molmasse aufweist als das Hydrogel auf Dextranbasis (2.000 g/mol gegenüber 500.000 g/mol) und dementsprechend eine sehr viel geringere physikalische Schichtdicke als diejenige mit Hydrogel.

## Patentansprüche

1. Einrichtung für die Durchführung und/oder den Nachweis von Affinitätsreaktionen, insbesondere Sondeneinrichtung, mit mindestens einer Oberfläche oder Grenzfläche, an der eine Affinitätsreaktion stattfindet, und mindestens einem an die Oberfläche gebundenen Polymer, **dadurch gekennzeichnet daß** die Bindung des Polymers an die Oberfläche in reiner Phase, d.h. im wesentlichen lösemittelfrei, erfolgt.

2. Einrichtung nach Anspruch 1, **dadurch gekennzeichnet, daß** es sich bei dem Polymer um ein weitgehend unverzweigtes, vorzugsweise kurzkettiges Polymer handelt.

3. Einrichtung nach Anspruch 1 oder Anspruch 2, **dadurch gekennzeichnet, daß** das Polymer eine Molmasse von 200 bis 50.000 g/mol, vorzugsweise von 200 bis 20.000 g/mol aufweist.

4. Einrichtung nach einem der vorhergehenden Ansprüche, **dadurch gekennzeichnet, daß** das Polymer einen Schmelzpunkt von < 200 °C, vorzugsweise von < 100 °C, besitzt.

5. Einrichtung nach einem der vorhergehenden Ansprüche, **dadurch gekennzeichnet, daß** das Polymer endständige funktionelle Gruppen besitzt, wobei es sich vorzugsweise um endständig homo- oder heterosubstituierte bifunktionelle Polymere handelt.

6. Einrichtung nach Anspruch 5, daß es sich bei den funktionellen Gruppen um NH₂-, OH-, SH-, COOH-, OCH₃-, Oxiran-, SCN- und/oder OCN-Gruppen handelt, wobei NH₂, OH- und/oder COOH-Gruppen bevorzugt sind.

7. Einrichtung nach einem der vorhergehenden Ansprüche, **dadurch gekennzeichnet, daß** es sich bei dem Polymer um Mischungen verschiedener Polymere oder um Copolymere handelt.

8. Einrichtung nach einem der vorhergehenden Ansprüche, **dadurch gekennzeichnet, daß** es sich bei dem Polymer um ein Polyalkylenglykol, insbesondere um Polyethylenglykol handelt.

9. Einrichtung nach einem der Ansprüche 1 bis 7, **dadurch gekennzeichnet, daß** es sich bei dem Polymer um ein Polyalkylenimin, insbesondere ein Polyethylenimin, handelt.

10. Einrichtung nach einem der vorhergehenden Ansprüche, **dadurch gekennzeichnet, daß** die Oberfläche an einem Träger oder Substrat ausgebildet ist.

11. Einrichtung nach einem der vorhergehenden Ansprüche, **dadurch gekennzeichnet, daß** die Oberfläche vor Anbindung des Polymers freie OH-Gruppen trägt.

12. Einrichtung nach einem der vorhergehenden Ansprüche, **dadurch gekennzeichnet, daß** die Oberfläche von einem Metall oder Metalloxid gebildet ist, wobei es sich bei dem Metalloxid vorzugsweise um Siliciumdioxid handelt.

13. Einrichtung nach einem der vorhergehenden Ansprüche, **dadurch gekennzeichnet, daß** die Oberfläche vor Anbindung des Polymers modifiziert oder aktiviert ist, wobei vorzugsweise zur Modifizierung/Aktivierung funktionelle Gruppen, insbesondere durch Silanisierung, eingeführt sind.

14. Einrichtung nach Anspruch 13, **dadurch gekennzeichnet, daß** es sich bei den funktionellen Gruppen um NH₂-, OH-, SH-, Ester-, Oxiran-, SCN- und/oder OCN-Gruppen handelt.

15. Einrichtung nach einem der vorhergehenden Ansprüche, **dadurch gekennzeichnet, daß** das Polymer als flüssiges oder geschmolzenes Polymer zur Bindung an die Oberfläche eingesetzt ist.

16. Einrichtung nach einem der vorhergehenden Ansprüche, **dadurch gekennzeichnet, daß** die Bindung des Polymers an die Oberfläche bei Temperaturen unter 200 °C, vorzugsweise zwischen 40 °C und 100 °C, insbesondere zwischen 50 °C und 80 °C, erfolgt.

17. Einrichtung nach einem der vorhergehenden Ansprüche, **dadurch gekennzeichnet, daß** die Bindung des Polymers an die Oberfläche innerhalb eines Zeitraums von einigen Stunden bis einigen Tagen erfolgt.

18. Einrichtung nach einem der vorhergehenden Ansprüche, **dadurch gekennzeichnet, daß** an dem an die Oberfläche gebundenen Polymer ein Ligand, insbesondere ein biochemisch aktiver Ligand immobilisiert ist, der für eine Affinitätsreaktion zur Verfügung steht.

19. Verfahren zur Herstellung einer oberflächenmodifizierten Einrichtung für die Durchführung und/oder den Nachweis von Affinitätsreaktionen, insbesondere zur Herstellung einer oberflächenmodifizierten Sonde, **dadurch gekennzeichnet, daß** mindestens ein Polymer an eine Oberfläche, an der eine Affinitätsreaktion stattfindet, in reiner Phase, d.h. im wesentlichen lösemittelfrei, gebunden wird.

20. Verfahren nach Anspruch 19 **gekennzeichnet durch** die Verwendung eines Polymers mit mindestens einem der Merkmale der Ansprüche 2 bis 9.

21. Verfahren nach Anspruch 19 oder Anspruch 20 **gekennzeichnet durch** die Verwendung einer Oberfläche mit mindestens einem der Merkmale der Ansprüche 10 bis 14.

22. Verfahren nach einem der Ansprüche 19 bis 21, **dadurch gekennzeichnet, daß** es gemäß mindestens einem der Merkmale der Ansprüche 15 bis 17 durchgeführt wird.

23. Verfahren nach einem der Ansprüche 19 bis 22, **dadurch gekennzeichnet, daß** an das Polymer ein Ligand, insbesondere ein biochemisch reaktiver Ligand, gekuppelt wird.

24. Verwendung der Einrichtung nach mindestens einem der Ansprüche 1 bis 18 zur markierungsfreien Detektion von Affinitätsreaktionen, insbesondere biochemischen Affinitätsreaktionen.

25. Verwendung nach Anspruch 24, **dadurch gekennzeichnet, daß** zur Detektion ein optisches Verfahren, vorzugsweise die reflektometrische Interferenzspektroskopie, eingesetzt wird.

## Claims

1. Device for carrying out and/or detecting affinity reactions, in particular probe device, with at least one surface or boundary surface on which an affinity reaction takes place, and at least one polymer bound to the surface, **characterised in that** the polymer is bound to the surface in its pure phase, i.e. essentially free of solvents..

2. Device according to claim 1, **characterised in that** the polymer is largely a linear, preferably short-warp, polymer.

3. Device according to claim 1 or claim 2, **characterised in that** the polymer comprises a molar mass of 200 to 50,000 g/mol, preferably of 200 to 20,000 g/mol.

4. Device according to one of the preceding claims, **characterised in that** the polymer has a melting point of < 200 °C, preferably of < 100 °C.

5. Device according to one of the preceding claims, **characterised in that** the polymer has terminal functional groups, these groups being preferably terminally homo- or heterosubstituted bifunctional polymers.

6. Device according to claim 5, **characterised in that** the functional groups are NH₂, OH, SH, COOH, OCH₃, oxirane, SCN and/or OCN groups, NH₂, OH and/or COOH groups being preferred.

7. Device according to one of the preceding claims, **characterised in that** the polymers are mixtures of various polymers or copolymers.

8. Device according to one of the preceding claims, **characterised in that** the polymer is a polyalkylene glycol, in particular a polyethylene glycol.

9. Device according to one of claims 1 to 7, **characterised in that** the polymer is a polyalkylene imine, in particular a polyethylene imine.

10. Device according to one of the preceding claims, **characterised in that** the surface is formed at a carrier or substrate.

11. Device according to one of the preceding claims, **characterised in that** the surface carries free OH groups before the bonding of the polymer.

12. Device according to one of the preceding claims, **characterised in that** the surface is made of a metal or a metal oxide, the metal oxide being preferably a silicon dioxide.

13. Device according to one of the preceding claims, **characterised in that** the surface is modified or activated before the polymer is bound to it, preferably functional groups being introduced for the modification/activation, in particular by silanization.

14. Device according to claim 13, **characterised in that** the functional groups are NH₂, OH, SH, ester, oxirane, SCN and/or OCN groups.

15. Device according to one of the preceding claims, **characterised in that** the polymer is employed as a liquid or molten polymer for the bonding at the surface.

16. Device according to one of the preceding claims, **characterised in that** the bonding of the polymer at the surface is effected at temperatures below 200 °C, preferably between 40 °C and 100 °C, in particular between 50 °C and 80 °C.

17. Device according to one of the preceding claims, **characterised in that** the bonding of the polymer at the surface is effected within a period of some hours to some days.

18. Device according to one of the preceding claims, **characterised in that** at the polymer bound to the surface a ligand, in particular a biochemically active ligand, is immobilised which is available for an affinity reaction.

19. Process for preparing a surface-modified device for carrying out and/or detecting affinity reactions, in particular for preparing a surface-modified probe, **characterised in that** at least one polymer in a pure phase, i.e. essentially free of solvents, is bound to a surface on which an affinity reaction takes place.

20. Process according to claim 19, **characterised by** the use of a polymer with at least one of the features of claims 2 to 9.

21. Process according to claim 19 or claim 20, **characterised by** the use of a surface with at least one of the features of claims 10 to 14.

22. Process according to one of claims 19 to 21, **characterised in that** it is carried out according to at least one feature of claims 15 to 17.

23. Process according to one of claims 19 to 22, **characterised in that** a ligand, in particular a biochemically reactive ligand, is coupled to the polymer.

24. Use of the device according to at least one of claims 1 to 18 for a detection of affinity reactions, in particular biochemical affinity reactions, without using markers.

25. Use according to claim 24, **characterised in that** for the detection an optical process, preferably the reflectometric interference spectroscopy, is employed.

## Revendications

1. Dispositif pour réaliser et/ou détecter des réactions d'affinité, en particulier dispositif à sonde présentant au moins une surface ou une surface limite au niveau de laquelle une réaction d'affinité se produit, et au moins un polymère lié à la surface, **caractérisé en ce que** la liaison du polymère à la surface se produit dans une phase pure, c'est à dire essentiellement exempte de solvant.

2. Dispositif selon la revendication 1, **caractérisé en ce qu'**il s'agit pour le polymère d'un polymère largement non ramifié, de préférence à chaîne courte.

3. Dispositif selon la revendication 1 ou la revendication 2, **caractérisé en ce que** le polymère possède une masse molaire comprise entre 200 et 50 000 g/mole, de préférence entre 200 et 20 000 g/mole.

4. Dispositif selon l'une des revendications précédentes, **caractérisé en ce que** le polymère possède un point de fusion <200 °C, de préférence < 100 °C.

5. Dispositif selon l'une des revendications précédentes, **caractérisé en ce que** le polymère possède des groupes fonctionnels terminaux, s'agissant de préférence de polymères bifonctionnels terminaux homo- ou hétérosubstitués.

6. Dispositif selon la revendication 5, **caractérisé en ce qu'**il s'agit, concernant les groupes fonctionnels, de groupes NH₂-, OH-, SH-, COOH-, OCH₃-, oxirane-, SCN- et/ou OCN-, les groupes NH₂-, OH- et/ou COOH- étant préférés.

7. Dispositif selon l'une des revendications précédentes, **caractérisé en ce qu'**il s'agit, concernant le polymère, de mélanges de différents polymères ou de copolymères.

8. Dispositif selon l'une des revendications précédentes, **caractérisé en ce qu'**il s'agit, concernant le polymère, d'un polyalkylèneglycol, en particulier de polyéthylèneglycol.

9. Dispositif selon l'une des revendications 1 à 7, **caractérisé en ce qu'**il s'agit, concernant le polymère, d'une polyalkylèneimine, en particulier d'une polyéthylèneimine.

10. Dispositif selon l'une des revendications précédentes, **caractérisé en ce que** la surface est conçue au niveau d'un support ou d'un substrat.

11. Dispositif selon l'une des revendications précédentes, **caractérisé en ce que** la surface porte des groupes OH- libres avant la liaison du polymère.

12. Dispositif selon l'une des revendications précédentes, **caractérisé en ce que** la surface est formée d'un métal ou d'un oxyde métallique, s'agissant de préférence, concernant l'oxyde métallique, de dioxyde de silicium.

13. Dispositif selon l'une des revendications précédentes, **caractérisé en ce que** la surface est modifiée ou activée avant la liaison du polymère, des groupes fonctionnels étant de préférence introduits à des fins de modification /d'activation, en particulier par silanisation.

14. Dispositif selon la revendication 13, **caractérisé en ce qu'**il s'agit, concernant les groupes fonctionnels, de groupes NH₂-, OH-, SH-, ester-, oxirane-, SCN- et/ou OCN-.

15. Dispositif selon l'une des revendications précédentes, **caractérisé en ce que** le polymère est utilisé en tant que polymère liquide ou fondu pour se lier à la surface.

16. Dispositif selon l'une des revendications précédentes, **caractérisé en ce que** la liaison du polymère à la surface se fait à des températures inférieures à 200 °C, de préférence comprises entre 40 °C et 100 °C, en particulier entre 50 °C et 80 °C.

17. Dispositif selon l'une des revendications précédentes, **caractérisé en ce que** la liaison du polymère à la surface se fait sur un intervalle de temps compris entre quelques heures et quelques jours.

18. Dispositif selon l'une des revendications précédentes, **caractérisé en ce qu'**est immobilisé au niveau du polymère lié à la surface un ligand, en particulier un ligand actif sur le plan biochimique, lequel est disponible pour la réaction d'affinité.

19. Procédé de préparation d'un dispositif avec surface modifiée pour la réalisation et/ou la détection de réactions d'affinité, en particulier pour la fabrication d'une sonde avec surface modifiée, **caractérisé en ce qu'**au moins un polymère est lié à une surface au niveau de laquelle une réaction d'affinité se produit, dans une phase pure, c'est à dire essentiellement exempte de solvant.

20. Procédé selon la revendication 19, **caractérisé par** l'utilisation d'un polymère présentant au moins une des caractéristiques des revendications 2 à 9.

21. Procédé selon la revendication 19 ou la revendication 20, **caractérisé par** l'utilisation d'une surface présentant au moins une des caractéristiques des revendications 10 à 14.

22. Procédé selon l'une des revendications 19 à 21, **caractérisé en ce qu'**il est réalisé selon au moins une des caractéristiques des revendications 15 à 17.

23. Procédé selon l'une des revendications 19 à 22, **caractérisé en ce qu'**au polymère est couplé un ligand, en particulier un ligand réactif sur le plan biochimique.

24. Utilisation du dispositif selon au moins l'une des revendications 1 à 18 pour la détection sans marquage de réactions d'affinité, en particulier de réactions d'affinité biochimiques.

25. Utilisation selon la revendication 24, **caractérisée en ce que** l'on utilise pour la détection un procédé optique, de préférence la spectroscopie d'interférence réflectométrique.
